# EUROPEAN PATENT SPECIFICATION

(11) **EP 2 068 614 B1**
(45) Date of publication and mention of the grant of the patent: **28.03.2012**
(21) Application number: 07834740.8
(22) Date of filing: 12.09.2007
(51) Int. Cl.: A01H 4/00, C12N 5/00, C12M 3/04

(54) **Method and device for micropropagation of plants**
Verfahren und Vorrichtung zur Mikrovermehrung von Pflanzen
Procédé et dispositif de micropropagation de plantes

(30) Priority: 14.09.2006 NO 20064203
(43) Date of publication of application: 17.06.2009
(73) Proprietor: Forsberg, Owe, 4021 Stavanger (NO)
(72) Inventor: Forsberg, Owe, 4021 Stavanger (NO)
(74) Representative: Håmsø Patentbyrå ANS
(86) International application number: PCT/NO2007/000321
(87) International publication number: WO 2008/033032

(56) References cited:
- US-A- 5 300 128
- US-B1- 6 251 826
- US-B2- 6 753 178
- DRAGO LORENZO ET AL: "Inhibition of in vitro growth of enteropathogens by new Lactobacillus isolates of human intestinal origin" FEMS MICROBIOLOGY LETTERS, vol. 153, no. 2, 1997, pages 455-463, XP002574279 ISSN: 0378-1097
- IRIANTO A ET AL: "Probiotics in aquaculture." JOURNAL OF FISH DISEASES, vol. 25, no. 11, November 2002 (2002-11), pages 633-642, XP002574341 ISSN: 0140-7775

## Description

This invention concerns micro-reproduction of plants. More particularly, it concerns micro-reproduction of plants in a closed, non-sterile space, in which pieces of plant tissue are placed on a surface having light, air, moisture and nutrition supplied thereto. The plant tissue is placed in a bio-incubator and is supplied probiotics in order to increase the plant growth and to reduce the growth of undesirable bacterial and fungal cultures. The invention also concerns a device for carrying out the invention.

In this connection, micro-reproduction of plants implies reproduction of plants by means of a so-called tissue culture system, in which the plant tissue is divided into relatively small pieces and is cultivated from these pieces of tissue. The plants growing up by means of this method are similar in the sense that they possess identical genes. This distinguishes the method from conventional cultivation of plants from seeds or cuttings having dissimilar genes amongst the individual plants.

Micro-reproduction of plants has gained a certain spread due to the possibilities provided for growing plants, in which desirable properties with respect to e.g. resistance against harmful micro-organisms may be maintained.

According to prior art, and due to undesirable growth of harmful bacteria and species of fungi, it is necessary to use sterile receptacles as well as sterile nutritive materials, which renders the cultivation method relatively work intensive and costly.

The advantages provided by the cultivation method nevertheless contribute to the cultivation method gaining an increasing application.

From cultivation of ordinary plants in open terrain it is known to use so-called probiotics to substantiate growth of a desirable bacterial culture, simultaneously inhibiting growth of undesirable cultures. Supposedly, probiotics function by taking up available resources so as to prevent harmful bacteria from multiplying.

As such, US patent No. 6,251,826 describes the application of a pulverized and sieved alfalfa into which humus and a wetting agent, among other things, has been added, which is particularly useful on grass lawns where the invention provides a favourable effect against common grass turf diseases.

Probiotics for use in open terrain is available from e.g. ABC ORGANICS INC. Casper, Wyoming, USA.

Probiotics of this type comprise naturally occurring lactic bacteria, for example: lactobacillus acidophilus, casel, plantarum, salivarius, bulgaricus, as well as photosynthetic bacteria, for example rhodobacter sphaeroides and rhodopseudomonas palustris, which is dosed in accordance with the manufacturer's instructions.

US 2002/0155595 provides a plant micropropagation apparatus and process in which a support platform for vessels contains a liquid growth media. The vessels are periodically pivoted to bring about an intermittent immersion of the plant tissue/growth substrate in the growth media.

US 5300128 discloses a method of plant tissue culture which comprises culturing a tissue or an organ of a plant, a part of the same or cultured cells in a medium containing a culture filtrate and/or an extract of a photosynthetic procaryotic microorganism.

Iritano A, et al: "Probiotics in aquaculture." Journal of fish diseases, vol. 25, no.11, November 2002 (2002-11), pages 633-642, XP002574341 ISSN 0140-7775 discusses the use of probiotics with respect to inhibiting fish pathogens.

The object of the invention is to remedy or reduce at least one of the disadvantages of the prior art.

The object is achieved according to the invention and by means of the features disclosed in the following description and in the subsequent claims.

A method in accordance with the invention for micro-reproduction of plants in a closed room, in which pieces of plant tissue are placed on a surface having at least light, air, moisture and nutrition supplied thereto, is characterized in placing the plant tissue in a bio-incubator and supplying probiotics containing at least lactic bacteria or photosyntetic bacteria thereto in order to increase the plant growth and to reduce the growth of undesirable bacterial and fungal cultures.

Advantageously, the probiotics form a part of a water mist supplied to the plant tissue. The losed room may be septic.

A bio-incubator in accordance with the invention comprises at least a cabinet with several shelves for plant tissue, a water mist generator arranged in a manner allowing it to produce and supply air mixed with water mist to the plant tissue, a circulation system for nutrients to the plant tissue, and also a required lighting rig for lighting of the plant tissue.

The shelves are comprised of cultivation drawers within which the plant material is placed on a growth surface at a distance from a drawer bottom of the cultivation drawer. Typically, the growth surface is coated with cloth or felt. If cloth is used, it should be watertight but not airtight. Superfluous nutrient mixture supplied to the growth surface is drained to the cultivation drawer bottom.

The circulation system for nutrients comprises a nutrient mixture receptacle, a monitoring system with a measuring sensor for nutrient content in the nutrient mixture, a dosing system for admixing of nutrients to the nutrient mixture, a pump communicating with the surface onto which the plant tissue is located, and a return path from the drawer and onwards to the nutrient mixture receptacle.

Advantageously, the measuring sensor in the cultivation drawer may be placed on the surface onto which the plant tissue is located. Typically, the measuring sensor measures moisture, acidity and concentration of nutrient salts.

The nutrient mixture, which typically comprises nutrient salts, growth hormones and probiotics, is not sterile, and it may be necessary to shield the nutrient mixture from too much light in order to prevent undesirable growth of algae.

The water mist generator, which communicates with the surface onto which plant tissue is placed, comprises a dosing apparatus for probiotics. As mentioned, moisture in the form of water mist is supplied to the plant tissue. Advantageously, water drops may be fragmented into a mist in an ultrasonic apparatus. An ultrasonic apparatus of the type FVA 400 made by Stultz GmbH, Hamburg, has proven suitable for the purpose.

Probiotics are added to the water in a desired concentration before supplying the water to the ultrasonic apparatus. Preferably, the supply of water mist is carried out in intervals and is adapted to the temperature and the light intensity.

Several cabinets, each of which comprises several shelves for plant tissue, are provided side by side, a lighting rig being provided between the cabinets. It may be necessary to place the bio-incubator in a temperature-regulated room.

The method according to the invention offers a significant improvement in efficiency of micro-reproduction cultivation methods owing to the fact that it is not necessary to use sterile growth receptacles and growth media. By means of the method according to the invention, micro-reproduction may be transferred from a laboratory scale operation to an industrial scale operation.

A non-limiting example of a preferred method and embodiment is described in the following and is depicted in the accompanying drawings, in which:

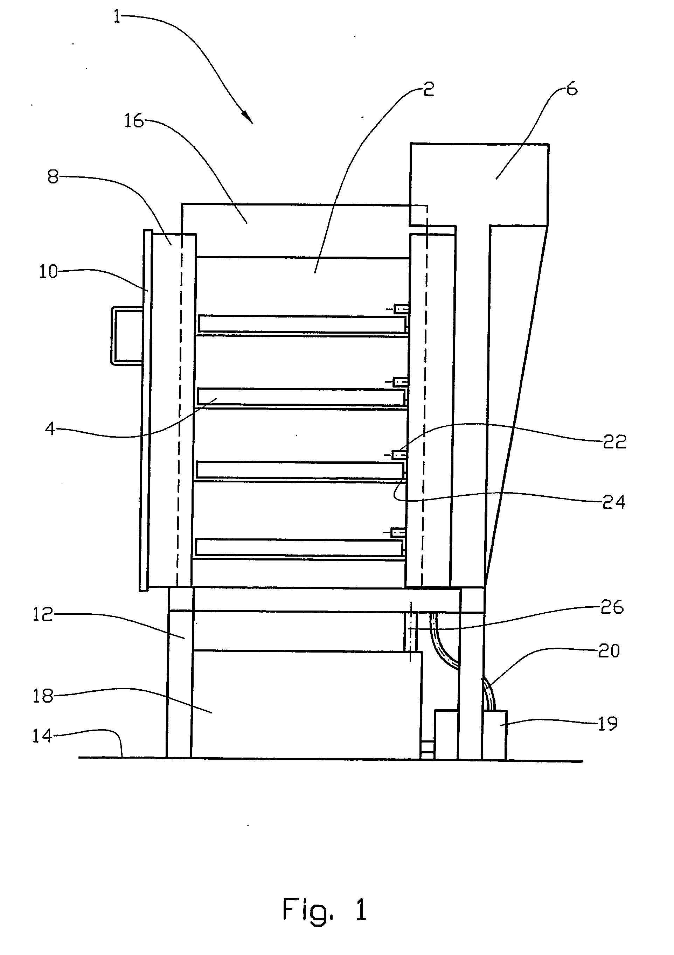

- Fig. 1: shows a side view of a bio-incubator within which only a lighting rig is shown;

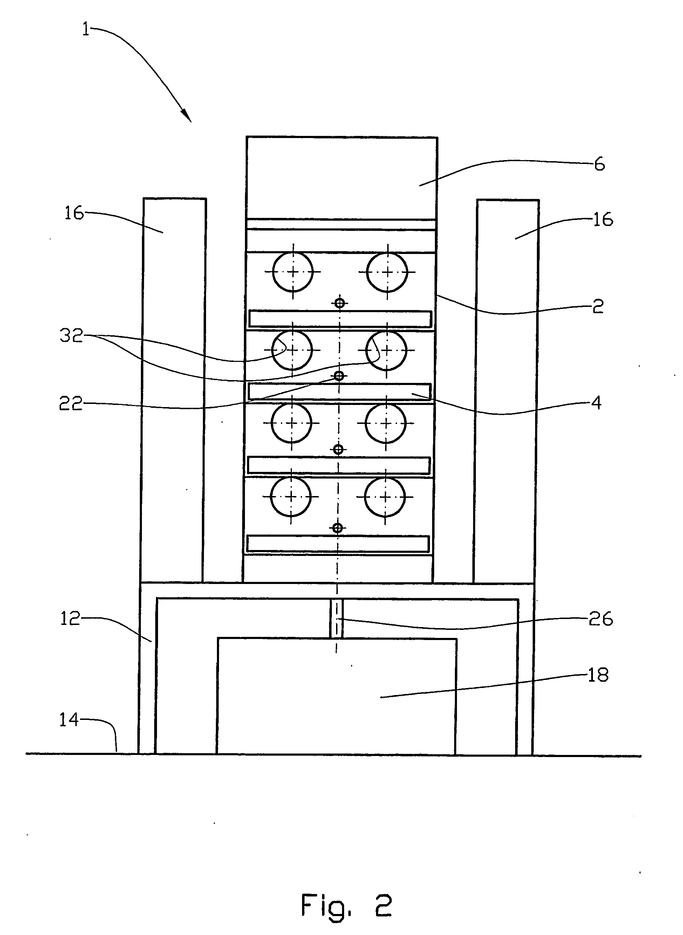

- Fig. 2: shows the bio-incubator of fig. 1 seen from the door side, the door of which is not shown;
- Fig. 3: shows, in perspective, a back piece of the bio-incubator, in which part of the covers have been removed for illustrative purposes; and

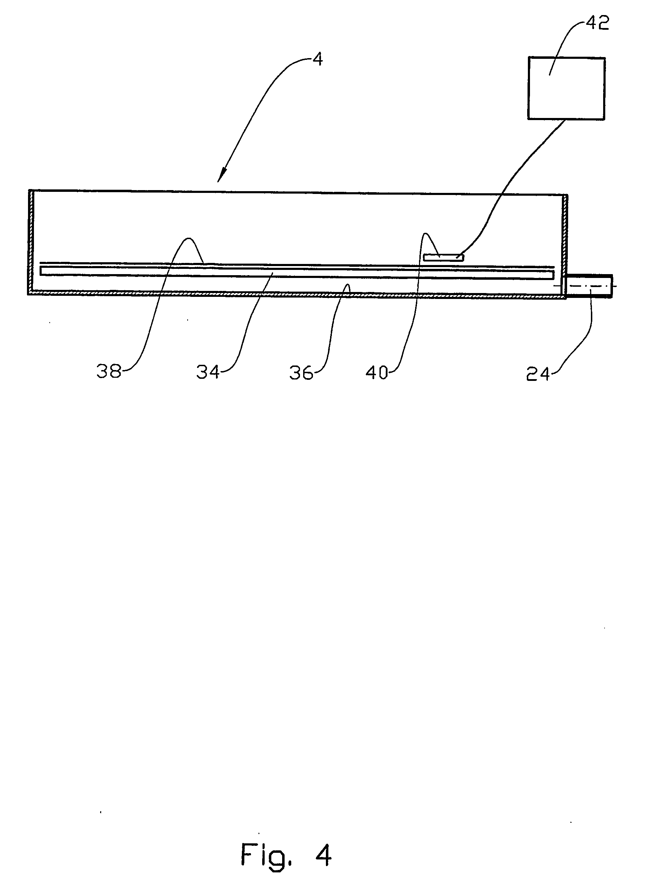

- Fig. 4: shows a vertical section of a drawer for cultivation of plant material.

In the drawings, reference numeral 1 denotes a bio-incubator comprising a transparent cabinet 2 having placed therein four cultivation drawers 4, a back piece 6 and a front housing 8 provided with a door 10. The door 10 may be opened to gain access to the cultivation drawers 4.

The bio-incubator 1 is placed on a stand 12 on a floor 14, the stand 12 also carrying a lighting rig 16 at each side of the cabinet 2. The lighting rig 16 is placed at a distance from the cabinet 2 in order to avoid unnecessary heating of the cabinet 2.

A nutrient receptacle 18 is placed underneath the cabinet 2, a pump 19 pumping a nutrient mixture from the nutrient receptacle 18, via a pipe 20 and onwards to dosing systems 22, typically in the form of valves for each cultivation drawer 4.

Surplus nutrient mixture is drained back into the nutrient receptacle 18 via drainage pipes 24 from the cultivation drawers 4 and a collecting pipe 26.

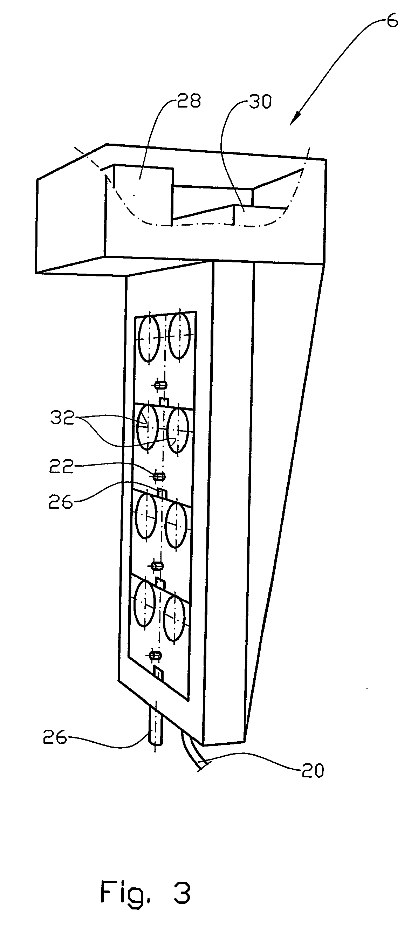

A water tank 28, which communicates with a water mist generator 30, see fig. 3, is provided in the back piece 6. The water mist generator 30 is optionally connected to water mist openings 32 in the back piece 6. Two water mist openings 32 in the back piece 6 correspond with each cultivation drawer 4.

Probiotics have been added in the water tank 28, whereby the water mist entering via the water mist openings 32 comprises a portion of probiotics.

The cultivation drawers 4 are formed with a growth plate 34 located at a distance from a bottom 36 of the cultivation drawer 4. A cloth or felt 38, onto which the plant material is disposed, is placed on the growth plate 34.

Nutrient mixture is supplied from the dosing system 22 and flows down onto the cloth or felt 38. Surplus nutrient mixture is drained from the cloth or felt 38 and down onto the bottom 36 of the cultivation drawer 4, from which the nutrient mixture flows back to the nutrient mixture receptacle 18, as mentioned.

A first sensor 40 is placed in the cultivation drawer and is arranged in a manner allowing it to measure moisture, acidity and concentration of nutrient salts. The first sensor 40 is connected to a programmable controller 42 which, via sensors not shown, measures light intensity and temperature in the cabinet 2 in a manner known per se. Based on predetermined values for said physical parameters, the programmable controller 42 controls the supply of nutrient mixture and water mist to each cultivation drawer 4, and also temperature and light intensity in the cabinet 2. The programmable controller 42 monitors the level and concentration in the nutrient mixture receptacle 18 and the water tank 28.

## Claims

1. A method for micro-reproduction of plants in a closed room, in which pieces of plant tissue are placed on a growth plate (34) having at least light, air, moisture and nutrition supplied thereto, **characterized in** placing the plant tissue in a bio-incubator (1) and supplying probiotics containing at least lactic bacteria or photosynthetic bacteria thereto in order to increase the plant growth and to reduce the growth of undesirable bacterial and fungal cultures.

2. The method according to claim 1, **characterized in** mixing probiotics containing at least lactic bacteria or photosynthetic bacteria into a water mist supplied to the plants.

3. The method according to claim 1, **characterized in that** the closed room is septic.

## Patentansprüche

1. Verfahren zur Mikroreproduktion von Pflanzen in einem geschlossenen Raum, in dem Teile von Pflanzengewebe auf eine Kulturplatte (34) gelegt werden, die dazu mindestens mit Licht, Luft, Feuchtigkeit und Nährstoffe versorgt werden, **dadurch gekennzeichnet, dass** das Pflanzengewebe in einen Bioinkubator (1) gelegt wird und probiotische Stämme beigefügt werden, die mindestens Milchsäurebakterien oder photosynthetische Bakterien enthalten, um das Pflanzenwachstum zu steigern und um das Wachstum von unerwünschten bakteriellen und Pilzkulturen zu mindern.

2. Verfahren nach Anspruch 1, **dadurch gekennzeichnet, dass** probiotische Kulturen, die mindestens Milchsäurebakterien oder photosynthetische Bakterien enthalten, in Wassernebel gemischt werden, der auf die Pflanzen aufgebracht wird.

3. Verfahren nach Anspruch 1, **dadurch gekennzeichnet, dass** der geschlossene Raum septisch ist.

## Revendications

1. Procédé de micro-propagation de plantes dans un espace fermé, dans lequel des morceaux de tissue de plantes sont placés sur une boîte de Petri (34), à laquelle sont fournis au moins de la lumière, de l'air, de l'humidité et des nutriments, **caractérisé en ce que** le tissue de plantes est disposé dans un incubateur biologique (1) et **en ce que** des probiotiques sont fournies, comprenant au moins de lactobacilliaceae et des bactéries photosynthétiques pour augmenter la croissance des plantes et pour réduire la croissance des cultures de bactéries et cryptogamiques.

2. Procédé selon la revendication 1, **caractérisé en ce que** les probiotiques, qui comprennent au moins des lactobcilliaceae ou des bactéries photosynthétiques, sont fournies dans une brume d'eau, qui est fournie aux plantes.

3. Procédé selon la revendication 1, **caractérisé en ce que** l'espace fermé est septique.
